Europäisches Patentamt

European Patent Office

Office européen des brevets

(11) Publication number: **0 128 743**
A2

# (12) EUROPEAN PATENT APPLICATION

(21) Application number: 84303833.2

(22) Date of filing: 06.06.84

(51) Int. Cl.³: **C 12 N 15/00**, C 12 P 21/02,
C 12 N 1/18, C 07 C 103/52
// (C12N1/18, C12R1/865)

---

(30) Priority: 07.06.83 US 502001

(43) Date of publication of application: 19.12.84
Bulletin 84/51

(84) Designated Contracting States: **AT BE CH DE FR GB IT LI LU NL SE**

(71) Applicant: **GENEX CORPORATION, 6110 Executive Boulevard, Rockville Maryland 20852 (US)**

(72) Inventor: **Strausberg, Robert Lawrence, 2815 Hathaway Terrace, Silver Spring Maryland (US)**
Inventor: **Strausberg, Susan Lee, 2815 Hathaway Terrace, Silver Spring Maryland (US)**

(74) Representative: **Watkins, Arnold Jack et al, European Patent Attorney Frank B. Dehn & Co. Imperial House 15-19 Kingsway, London WC2B 6UZ (GB)**

---

(54) Expression of foreign genes in yeast.

(57) A method of expressing foreign genes in yeast comprising transformation of yeast cells by introduction therein of a cloning vector containing a foreign gene, and yeast galactokinase gene regulatory region and promoter in position to regulate the expression of the foreign gene, and cultivating said transformed yeast cells in a nutrient medium under protein-producing conditions is disclosed. Suitable plasmids and microorganisms useful in the process are also disclosed. The method has been used to produce a GAL 1 - bovine calf prochymosin fusion protein in Saccharomyces cerevisiae.

EP 0 128 743 A2

EXPRESSION OF FOREIGN GENES IN YEAST

The present invention relates to a method of expressing foreign genes in yeast. The invention further relates to use of the yeast GAL 1 promoter and regulatory region to control foreign gene expression in yeast, vectors containing a foreign gene and the yeast GAL 1 promoter and regulatory region, yeast cells transformed by vectors containing a foreign gene and the GAL 1 promoter and regulatory region, and foreign gene products produced by yeast cells transformed by vectors containing a foreign gene and the GAL 1 promoter and regulatory region.

Description of the Background Art

While there has been a great deal of technical success in expressing foreign genes in procaryotic organisms, particularly Escherichia coli, there is an increasing interest in use of the yeasts, such as Saccharomyces cerevisiae and related yeasts, as alternative hosts for manufacturing mammalian and other foreign proteins. This interest has been stimulated in part by the extensive knowledge of yeast fermentation processes, and the safety of S. cerevisiae (generally regarded as safe by the U.S. Food and Drug Administration). S. cerevisiae has a well-defined genetic system (see, e.g., Mortimer, R.K. et al., In: The Molecular Biology of the Yeast Saccharomyces, Life Cycle and Inheritance, Strathern, Jones and Broach, eds.

New York: Cold Spring Harbor Laboratory, pp. 11-26 [1981]). Recently a transformation protocol and stable plasmid vectors have been reported (see, e.g., Hinnen, A. et al., Proc. Natl. Acad. Sci. USA, 75: 1929 [1978]).

Plasmids which have been developed for yeast transformation studies generally have several properties in common (Reviewed by Botstein, D. and Davis, R.W., In: The Molecular Biology of the Yeast Saccharomyces, Metabolism and Gene Expression, Strathern, Jones and Broach, eds., New York: Cold Spring Harbor Laboratory [1982]). Each have at least a portion of the E. coli pBR322 plasmid including the origin of replication and the ampicillin and/or tetracycline resistance genes. Inclusion of these genetic elements permits plasmid replication and selection in E. coli. In addition, these plasmids include a selectable marker to permit identification of yeast transformants. Markers for this purpose include the yeast TRP, LEU 2 and URA 3 genes. A yeast strain chosen for tranformation is generally deficient in the enzymatic function encoded by one or more of these genes, so that selection is based on the cloned yeast marker providing that function to permit cellular growth without the added metabolite. In addition, some of the yeast cloning vectors contain an origin of DNA replication to permit plasmid replication in yeast cells. These vectors are referred to as YRp if a yeast nuclear origin of replication (a.r.s.) is provided and YEp if the origin of replication from the natural yeast plasmid (the "2μ circle") is utilized. These vectors are termed shuttle vectors based on their ability to replicate in E. coli and yeast cells. Other vectors without a yeast origin of replication, termed YIp, can be used to transform yeast, and the plasmid genetic information can be stably maintained in yeast provided that a recombination event involving insertion of the plasmid DNA into a pre-existing yeast chromosome occurs.

An example of a useful yeast-E. coli shuttle vector of the YEp type is YEp13 (Broach, J.R., et al., Gene. 8: 121 [1979]). This plasmid includes the entire pBR322 sequence, the yeast 2 μ plasmid origin of replication, and the S. cerevisiae LEU 2 gene. Specific yeast genes have been isolated using the plasmid YEp13 in conjunction with transformation in yeast, by insertion of yeast DNA fragments into the plasmid. See Broach, J. R., et al., supra.

There has been considerable interest in the galactokinase gene of yeast as well. Galactokinase is one of four inducible enzymes required for galactose utilization by Saccharomyces, and is specified by the gene GAL 1. Galactokinase has been purified from cells of S. cerevisiae which were fully induced for the production of galactose metabolizing enzymes. See Schell, M.A. and Wilson, D.B., J. Biol. Chem., 252: 1162, (1977). These authors concluded that galactokinase is a prominant enzyme in the yeast cell, comprising up to 1.5% of the total cellular protein.

The galactose metabolic genes are coordinately regulated, and recently a model has been elucidated, based on genetic and biochemical evidence, to explain this tightly controlled gene expression. The essential components of the model include the following features. Each of the genes encoding the galactose metabolic enzymes have their own promoter regions and the regulation of these genes is at the transcriptional level. The transcriptional control is enforced by the protein products of the GAL 4 and GAL 80 genes. Each of these proteins are produced constitutively and they have antagonistic effects. According to the model, the GAL 4 product is a positive regulator of transcription and must bind in the regulatory region adjacent to the galactose promoters to induce transcription. When galactose is not present, the GAL 4 protein forms a complex with the GAL 80 protein. This association prevents the GAL 4 protein from inducing transcription of the galactose metabolic genes.

When galactose is present in the cell, association of the GAL 4 and GAL 80 proteins is inhibited and the GAL 4 protein is free to induce transcription. The entire control system can be circumvented if a simple carbon source such as glucose is also available for cellular utilization. In that case expression of the galactose metabolic genes is inhibited, even if galactose is present in the growth medium. The effect is referred to as glucose repression. A review of the regulation of the yeast galactose genes has recently been published (see Oshima, Y., In: The Molecular Biology of the Yeast Saccharomyces, Metabolism and Gene Expression, (1982) supra, pp 159-180 [1982]).

The galactokinase gene of S. cerevisiae (GAL 1) has recently been cloned by inserting yeast library DNA into the E. coli plasmid pBR322 and transforming E. coli cells which have a defective bacterial galactokinase gene (see Citron, B.A. et al., Gene, 6: 251 [1979] and Schell, M.A. et al., Gene, 5: 291 [1979]). It was observed that some of the cloned yeast DNA could be expressed in E. coli and that certain clones contained genetic information which provided the galactokinase function in E. coli. Subsequent analysis including DNA sequencing, revealed that the cloned DNA was the yeast galactokinase gene, and that some of these clones included the regulatory region between the GAL 1 and GAL 10 genes.

It is an object of the present invention to provide a method of expressing foreign genes in yeast utilizing the GAL 1 promoter and regulatory region.

In accordance with the present invention, a method of expressing foreign genes in yeast comprises transformation of yeast cells through the introduction therein of a cloning vector containing a foreign gene, and a yeast galactokinase gene regulatory region and promoter in a position to regulate the expression of the foreign gene, and cultivating such transformed cells in a nutrient medium under protein-producing condition.

BRIEF DESCRIPTION OF THE DRAWINGS

Figure 1:  A schematic diagram of plasmid YEp13.  The thin line indicates pBR322 sequences, the heavy line indicates pYeleu10 sequences, and the hatched line indicates 2 micron circle sequences.

Figure 2:  A schematic diagram of plasmid YpGX1.  This plasmid is a YEp13 plasmid with a Sal I site removed.

Figure 3:  A diagram showing the sequence of insertions of a foreign gene and the GAL 1 promoter and regulatory region into plasmid YpGX1.

Figure 4:  A schematic diagram of plasmid YpGX3, containing the bovine calf chymosin gene modified by insertion of a SAL I linker at the second codon position.

Figure 5:  A schematic diagram of plasmid YpGX4, containing the GAL 1 promoter and regulatory region in position to control the prochymosin gene.

DETAILED DESCRIPTION OF THE INVENTION

The procedures used to express foreign genes in yeast described herein are, except where otherwise indicated, accomplished by using conventional techniques of molecular biology, and can be divided into the following major stages, each of which is described more fully herein: (1) preparation of a foreign gene, GAL 1 promoter and regulatory region, and a cloning vector in a form suitable for transformation of yeast cells, (2) insertion of the foreign gene and GAL 1 promoter and regulatory region into the cloning vector, (3) transformation of yeast cells by the cloning vector containing the foreign gene and the GAL 1 promoter and regulatory region, and (4) cultivating such transformed cells in a nutrient medium under conditions suitable for producing the heterologous protein.

Initial cloning vector constructions are preferably screened by transforming the procaryotic organism Escherichia coli. It is preferred to utilize this organism in the preliminary stages because of the ease of transforming this procaryote and the simplicity in reisolating the vector DNA. It will be appreciated, however, that preliminary cloning can be conducted in other gram negative or gram positive bacteria, or such cloning may be accomplished using yeast cells. Cloning vectors may be plasmids or phages, with plasmids being preferred. The criteria utilized in developing appropriate cloning vectors according to the present invention include its ability to replicate in both E. coli and yeast, the presence of selectable markers for both systems, and the presence of suitable insertion sites for the GAL 1 promoter and regulatory region as well as the foreign gene.

The plasmid YEp13, Broach, J.R., et al., supra, meets most of the above criteria. The plasmid is a hybrid with portions originating from both E. coli and S. cerevisiae plasmids. The plasmid contains in part the yeast plasmid 2 micron circle which has a yeast origin of replication permitting vector replication in yeast and an S. cerevisiae transcription terminator (derived from the "Able" gene).

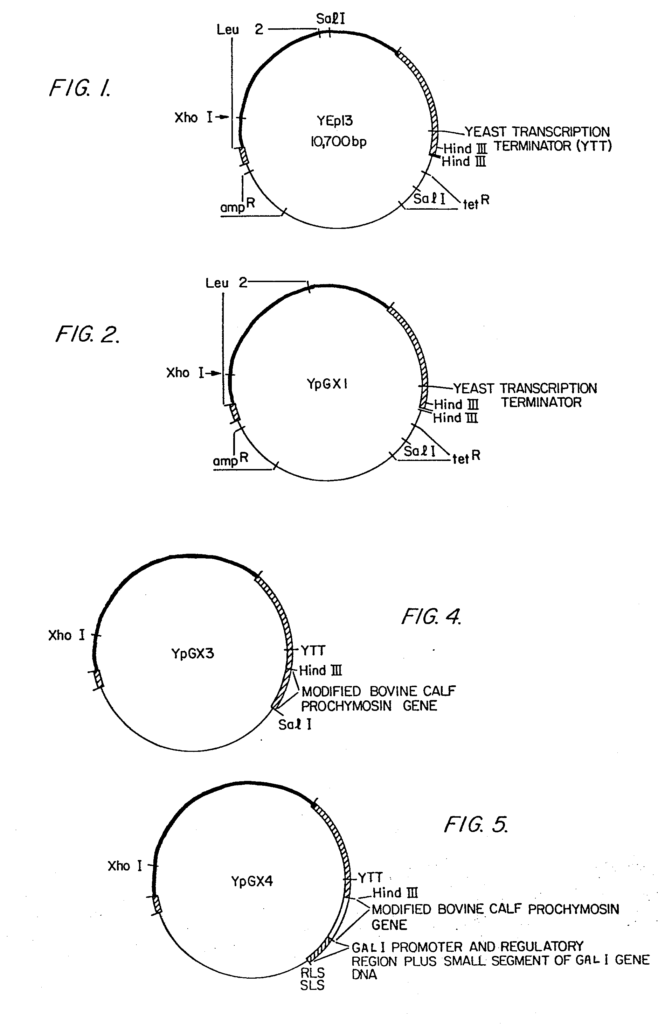

YEp13 also contains the entire bacterial plasmid pBR322, Bolivar, F., et al., Gene, 2, 95 (1977), which carries genes for ampicillin (amp) and tetracycline (tet) resistance. The YEp13 plasmid contains PvuII, Bam HI, and Hind III restriction endonuclease sites which can be easily utilized for DNA insertion. The plasmid YEp13 contains a portion of the plasmid pYeleu10 as well, which carries the entire yeast gene LEU 2, permitting plasmid selection in Leu⁻ yeast strains. A schematic drawing of plasmid YEp13 is provided in Figure 1.

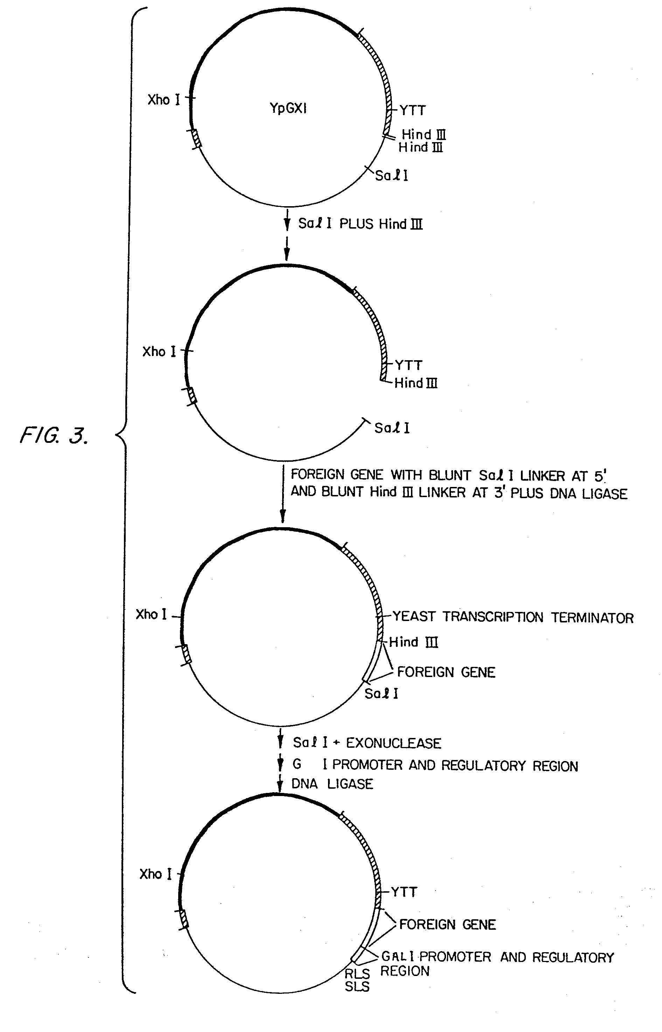

To facilitate the use of YEp13 as a cloning vector according to the present invention, the Sal I restriction endonuclease site adjacent to the yeast LEU 2 gene is eliminated by partial digestion with Sal I, removal of the resultant single strand DNA overhang with an appropriate exonuclease, and then blunt end ligated to reconstruct a circular plasmid. This operation results in a unique plasmid Sal I restriction endonuclease site, advantageously present in the tet gene. Plasmids which have undergone this modification are designated YpGX1 and a schematic drawing of this plasmid is provided in Figure 2.

A suitable amp⁻, tet⁻, strain of E. coli may be transformed with the plasmid YpGX1 using conventional methods (see, e.g., Lederberg, J. Bacteriology, 119: 1072 [1974]). Transformants are typically grown on a standard L-broth, containing ampicillin. Because that portion of the plasmid YpGX1 derived from the plasmid pBR322 imparts ampicillin resistance to the cells, colonies growing on the ampicillin-containing medium must contain the plasmid YpGX1, and are selected for further treatment.

Cells transformed with plasmid DNA which has undergone the above Sal I site removal procedure are again typically grown on a standard L-broth, containing ampicillin. Samples of colonies growing on the ampicillin-containing medium are then transferred to a second medium containing tetracycline.

Since removal of the Sal I site located within the tet gene of the plasmid would make the yeast cell tetracycline sensitive, only those colonies which are tet$^R$, amp$^R$ contain the plasmid YpGX1, and are selected for further treatment. The plasmid YpGX1 is in a form suitable for insertion of a foreign gene according to the present invention.

A foreign gene suitable for use according to the present invention advantageously contains a unique restriction site at the 5' end of the gene and another unique restriction site at or downstream from the 3' end of the gene. Foreign genes not meeting the above specifications, for example, bovine calf prochymosin (prorennin), may be altered using conventional methods of molecular biology to achieve the desired sequences.

A suitable vector according to the present invention is treated with appropriate restriction endonucleases to cleave the vector at the unique restriction sites. Copies of a foreign gene in suitable form as indicated above are treated with appropriate restriction endonucleases, introduced into a vector cleaved as described above, and ligated to the linearized vector DNA. The resulting plasmid is used to transform E. coli cells.

Since YpGX1 contains unique restriction sites Pvu II, Bam HI, Sal I and two adjacent Hind III sites, several modifications of the foreign gene would permit its insertion in an orientation so that transcription of that gene is oriented toward the "Able" gene transcription terminator. Other unique sites could be placed in the vector by ligating "linker" DNA molecules with linearized plasmid DNA.

In a particularly preferred embodiment, the foreign gene contains a unique Sal I restriction site at the 5' end and a unique Hind III site at the 3' end, or is so altered. Plasmids constructed as described above contain the foreign gene oriented within the plasmid so that the yeast transcription terminator is located just downstream of the foreign gene (see Figure 3).

Vectors constructed as described above are used to transform E. coli, and transformants are again grown on a standard L-broth, containing ampicillin. Samples of colonies growing on the ampicillin-containing medium are then transferred to a second medium containing tetracycline. Since the tetracycline resistance of the YpGX1 plasmid is destroyed by insertion of the foreign gene, only amp$^R$, tet$^S$ colonies are selected for further treatment.

Plasmids containing the foreign gene are capable of replication in yeast since the yeast origin of replication within the remaining portion of the plasmid derived from the 2 micron circle is still intact. In order to achieve expression of the foreign gene in yeast, the plasmid is preferably altered by insertion of the GAL 1 gene regulatory region and promoter from Saccharomyces carlsbergensis prior to transformation of yeast cells using the plasmid.

The GAL 1 promoter and regulatory region of S. carlsbergensis is preferred, since it is a high efficiency promoter, expression determined by this promoter is easily controlled by the presence (induced) or absence (non-induced) of galactose in the growth medium, and it has restriction endonuclease sites which facilitate ligation with foreign genes. The GAL 1 promoter and regulatory region is isolated from a Gal gene cluster by treatment with the enzyme Rsa I to isolate a segment containing the GAL 1 promoter and regulatory region plus a small portion of the GAL 1 gene coding for several amino acids.

The plasmid DNA with the foreign gene present is preferably treated with Sal I to open the plasmid. The resulting single strand DNA overhang is then preferably removed with mung bean exonuclease leaving blunt ends. Into this DNA is introduced the isolated segments containing the GAL 1 promoter and regulatory region plus the small portion of DNA derived from the GAL 1 gene. The segments are blunt end ligated to the linear plasmid DNA using DNA ligase, resulting in a circular DNA molecule (see Figure 3).

Plasmids containing the foreign gene and the GAL 1 promoter and regulatory region are preferably amplified in E. coli, extracted, and used to transform appropriate Leu⁻ strains of yeast according to the following procedure. The yeast cells are treated with the enzyme zymolyase to remove a portion of the yeast cell wall. The plasmids are mixed with the cells and sorbitol is added to the medium to prevent cell breakdown. The cells are then embedded in a medium containing sorbitol and agar to allow the yeast cells to regenerate their cell walls.

After regeneration of the cell walls, transformed yeast cells are grown on a minimal medium free of leucine. Because the cloning vector contains the LEU 2 gene, colonies growing on the leucine free medium contain that plasmid.

To identify a smaller group of colonies which contain the GAL 1 promoter and regulatory region oriented in the proper direction to regulate expression of the foreign gene, an immunological assay is preferably performed to determine which colonies contain cells which produce the foreign protein.

A particularly preferred immunological assay for use acccording to the present invention is the "Western" Blot analysis (see Burnette, W.N., A. Anal. Biochem., 112: 195 [1981]; and Towbin, H. et al., Proc. Natl. Acad. Sci. USA, 76: 4350 [1979]). This assay can be performed quantitatively and provides information concerning the completeness and stability of the foreign protein.

The immunological assay involves isolating total yeast cell protein from samples of each colony and allowing the proteins to separate on SDS polyacrylamide gel based on their molecular weights. After separation, the proteins are transferred to nitrocellulose paper and contacted with labeled antibodies to the foreign protein. Unbound antibodies are removed from the paper and bound antibodies are detected by their label (e.g., conjugated radiolabeled peroxidase). Antibody reaction demonstrates the presence of the foreign protein and indicates which transformants contain the GAL 1 promoter and regulatory region properly oriented for expression of the foreign gene.

The foreign protein produced by the transformants is a fusion protein containing the complete amino acid sequence coded for by the foreign gene plus several amino acids which are present as a result of the small segment of GAL 1 gene DNA located between the foreign gene and the GAL 1 regulatory region and promoter. Foreign proteins present in microbial cells are sometimes broken down by proteases produced by the cell. However, the fusion proteins produced by the present invention have generally been found not to be broken down by the yeast proteases.

Transformed yeast cells capable of fusion protein production are cultivated in a medium containing galactose to induce the GAL 1 promoter and allow expression of the foreign gene controlled by it. The medium is preferably leucine free so that the transformed cells will be forced to retain the plasmids, and glucose free to prevent glucose repression of the GAL 1 promoter.

These two requirements can be circumvented as described in Examples VIII and IX by integrating the genetic construction into a pre-existing yeast chromosome and by selecting for mutants in which galactokinose expression is constitutive and insensitive to glucose repression.

A major advantage in utilizing the GAL 1 promoter to direct expression of foreign genes is the well defined genetic system governing expression from this promoter. This system can be exploited to provide regulated or non-regulated expression and may be manipulated to enhance transcription levels.

Modification of this system to provide non-regulated expression, which has the advantage that an economical carbon source such as glucose can be used throughout the fermentation, is obtained by modifying the nuclear background of the host strain rather than altering vector DNA. Since regulation of the galactose metabolic system apparently involves an interaction between a positive element, the GAL 4 protein, and a negative regulator, GAL 80, constitutive expression can be obtained by altering either of these elements. In the case of GAL 4, a lesion termed GAL 81 apparently causes an inability of the GAL 4 protein to bind GAL 80 protein. Therefore, the GAL 81 allel protein can bind with the GAL 1 regulatory DNA without galactose in the cell. The same phenotype is observed if a lesion occurs in GAL 80. Presumably in that case, the GAL 80 protein loses the ability to bind the GAL 4 protein.

In the presence of GAL 81 or GAL 80, the galactose constitutive phenotype results, but expression is still inhibited by glucose in the growth medium. Mutants resistant to glucose repression have also been isolated and are described with respect to the present invention in Example VIII.

Increased copy number of the GAL 4 gene in the cell has been reported to enhance messenger RNA levels of the galactose metabolic genes in yeast (See Laughon, A. et al., Proc. Natl. Acad. Sci. USA, 79: 6827-6831 [1982]). This may be accomplished according to the present invention by isolating the GAL 4 gene from yeast DNA, and inserting it into YpGX1 using conventional methods (see, e.g., Laughon, A. et al., supra). For example, the GAL 4 gene from S. cerevisiae strain 95-4A may be isolated by partial digestion of 95-4A DNA with Sau 3A endonuclease. The isolated DNA fragment may then be ligated into the single BAM HI site of YpGX1 and cloned. The cloned GAL 4 gene may later be inserted into the unique PvuII site of YpGX4 as described in Example VI.

One embodiment of the present invention includes, as an additional step, linear integration of the circular plasmid into the yeast chromosome after transformation of the yeast cell. The Leu⁻ strain of yeast described above contains two mutations in the LEU 2 gene of the yeast chromosome which makes the gene non-functional. Since the cloning vector used in the present invention contains a functional yeast LEU 2 gene, there is sufficient homology between the mutant host chromosome LEU 2 gene and the LEU 2 gene of the plasmid for recombination to occur. The recombination event results in the linear integration of the plasmid DNA into the host chromosome in the region of homology. The host becomes Leu⁺ and the entire nucleotide sequence of the GAL 1 gene regulatory region and promoter as well as the foreign gene (and the GAL 4 gene if present) become a functional part of the yeast chromosome. This results in a stable system which can be grown on a medium containing leucine since there is no longer a concern that the plasmid will be lost from the host. Successful recombinants are selected by screening for those cells which have a functional LEU 2 gene within the host chromosome using conventional methods.

In a preferred embodiment of the present invention, the foreign gene contains the nucleotide sequence coding for bovine calf prochymosin (prorennin). It is

14

particularly preferred that the prorennin sequence be modified by the insertion of a Sal I linker at the second codon position as described in Example II.

The invention is further illustrated by reference to the following examples, which are not intended to be limiting.

## Example I
### Construction of the Plasmid YpGX1

YEp13 plasmids were introduced into E. coli strain HB101 using conventional methods (see, e.g., Lederberg and Cohen, J. Bact. 119: 1072 [1974]). Transformants were grown on standard LB-ampicillin (30 μg/ml) plates at 37 degrees C for 24 hours. Only ampicillin resistant colonies were selected for further treatment.

YEp13 DNA was partially digested with restriction endonuclease Sal I. The DNA was then treated with mung bean exonuclease, and thereafter treated with DNA ligase to recircularize the plasmids. The transformed cells were then grown on standard LB-ampicillin (30 μg/ml) plates at 37 degrees C for 24 hours. Colonies were picked to LB-tetracycline (10 μg/ml) plates to determine phenotype. Only tetracycline resistant colonies were selected for further treatment. Approximately twenty clones were isolated. The desired plasmid was designated YpGX1 (Figure 2).

## Example II
### Construction of the Plasmid pGX344

The plasmid pGX1225 (deposited with the Northern Regional Research Laboratories, Peoria, Illinois NRRL B-15061) (plasmid pBR322 carrying the cloned prochymosin (prorennin) cDNA sequence in the ampicillin gene) was linearized by restriction endonuclease digestion at a concentration of 50 $\mu$g/ml in 6mM Tris-HC1 (pH 7.4), 150 mM NaC1, 6mM MgC1$_2$, 6mM $\beta$-mercaptoethanol, 100 $\mu$g/ml BSA, and 50 U/ml PvuI for 1 hour at 37 degrees C. Digestion was determined by incubation for 7 minutes at 65 degrees C and was determined to be complete by agarose gel electrophoresis.

The linearized plasmid was resected at a concentration of 5 $\mu$g/ml in 20 mM Tris-HC1 (pH 8.1), 200 mM NaC1, 12 mM MgC1$_2$, 12 mM CaC1$_2$, 1 mM EDTA, and 5 U/ml Bal 31 for 90 seconds at 30 degrees C. Resection was terminated by addition of EDTA to a final concentration of 35 mM and incubation on ice for 5 minutes. The DNA was purified and concentrated by phenol/chloroform extraction and ethanol precipitation.

The resected DNA was blunted at a concentration of 40 $\mu$g/ml in 50 mM Tris-HC1 (pH 7.8), 10 mM MgC1$_2$, 20 mM DTT, 50 ug/ml BSA, 20 $\mu$M dATP, dCTP, dGTP, dTTP, and 15 U/ml DNA Polymerase I (large fragment) for 1 hour at 12 degrees C. Blunting was terminated by incubation for 7 minutes at 65 degrees C.

Sal I linkers were added and vector DNA was ligated into circles in the same reaction. Blunt DNA at a concentration of 7 $\mu$g/ml and 7 $\mu$g/ml Sal I linkers, kinased by conventional methods (see, e.g., Maxam and Gilbert, PNAS 74: 560, [1977]), were added to 50 mM Tris-HCL (pH 7.8), 10 mM MgC1$_2$, 20 mM DTT, 50 $\mu$g/ml BSA, 1 mM ATP, and 8 x 10$^3$ U/ml T4 DNA ligase for 24 hours at 12 degrees C.

E. coli HB101 competent cells, prepared using conventional methods (see, e.g., Mandel and Higa, J. Mol. Biol. 53: 154 [1970]) were transformed with 400 ng ligated DNA per ml of cells. Colonies were selected on standard L-broth plates containing 15 $\mu$g/ml tetracycline. Plasmid DNA from individual transformant colonies were prepared by conventional methods (see, e.g., Holmes and Quigley, Anal.

Biochem. 14: 193 [1981]). Analytical restriction endonuclease digestions were done on clones containing two Sal I sites. Such clones were digested with various enzymes and electrophoretic fragment sizes calculated to determine the extent of Bal31 resection.

The plasmid pGX334 was selected as a prochymosin (prorennin) resected clone and pure plasmid DNA from an isolated single colony prepared using conventional methods (see, e.g., Clewell and Halinski, J. Bacteriol. 110: 1135 [1972]) was sequenced (see, e.g., Maxam and Gilbert, supra). The added Sal I linker was found to be in the second codon of the prochymosin (prorennin) coding sequence.

The rennin (chymosin) coding region of pGX334 was reconstructed in pGX323 (final PstI fragment of rennin cloned into the PstI site of the M13 sequence) by transfer of SalI/BglII fragment. Pure plasmid was sequentially digested with KpnI, BglII, SalI for pGX323 and KpnI, SalI, EcoRI for pGX334. Digestion was determined to be complete by gel electrophoresis. Phenol/chloroform extraction and ethanol precipitation were used to purify and concentrate the DNA.

Plasmid pGX334/KpnI/SalI/EcoRI at a concentration of 33 ug/ml and plasmid pGX323/KpnI/BglII/SalI at a concentration of 100 ug/ml were ligated in 50mM Tris-HCl (pH 7.8), 10mM $MgCl_2$, 20 mM DTT, 50 ug/ml BSA, 1mM ATP, and $13 \times 10^3$ U/ml T4 DNA ligase for 24 hr at 12° C.

E. coli HB101 competent cells prepared by conventional methods (see. e.g., Mandel and Higa, supra) were transformed with 1 ug ligated DNA/ml of cells. Colonies were selected on standard L-broth containing 30 ug/ml ampicillin. Plasmid DNA from individual transformant colonies was isolated by conventional methods (see, e.g., Holmes and Quigley, supra). EcoRI and SalI analytical digests were used to identify the clone bearing the complete prorennin with a SalI site in the second codon ·. The desired plasmid is termed pGX344.

## Example III
### Construction of the Plasmid YpGX3

Plasmid pGX344 was linearized by restriction endonuclease digestion at a concentration of 160 μg/ml in 50 mM NaC1, 10 mM Tris (pH 7.4), 10 mM MgSO$_4$, 0.1 mM DTT, 100 μg/ml BSA, and 200 units/ml HindIII (New England Biolabs) for two hours at 37° C. Digestion was terminated by incubation for 5 minutes at 65° C and was determined to be complete by agarose gel electrophoresis.

The linearized DNA was digested with restriction endonuclease SalI at a concentration of 160 μg/ml in 100 μg/ml BSA (bovine serum albumin), and 60 units/ml SalI (Boehringer Mannheim) for two hours at 37° C and examined for completion by agarose gel electrophoresis. The reaction was terminated by heating at 65 degrees C for 5 minutes. The small SalI-HindIII fragment (approximately 1200 base pairs) was isolated by agarose gel electrophoresis and electroelution. This fragment will subsequently be termed "fragment A".

YpGX1 was linearized by restriction endonuclease digestion at a concentration of 50 μg/ml in 50 mM NaC1, 10 mM Tris (pH 7.4), 10 mM MgSO$_4$, 0.1 mM DTT, 100 μg/ml BSA, 200 units/ml HindIII (New England Biolabs) for 2 hours at 37 degrees C. Digestion was terminated by incubation for 5 minutes at 65 degrees C and was determined to be complete by agarose gel electrophoresis.

The linearized DNA was digested with SalI at a concentration of 50 μg/ml in 100 mM NaC1, 50 mM Tris (pH 7.4), 10 mM MgSO$_4$, 0.1 mM DTT, 100 μg/ml BSA, 60 units/ml SalI (Boehringer Mannheim) for two hours at 37° C and examined for completion by agarose gel electrophoresis.

The reaction was terminated by heating at 65° C for 5 minutes.

The large fragment (approximately 10,000 bp) was isolated by electroelution following agarose gel electrophoresis. This DNA molecule is termed "fragment B". Fragments A and B were ligated at a concentration of 33 µg/ml fragment A, 83 µg/ml fragment B, 50 mM Tris-HCL (pH 7.8), 10 mM $MgCl_2$, 20 mM DTT, 50 µg/ml BSA, 1 mM ATP and 1 x $10^3$ units/ml T4 DNA ligase (New England Biolabs) at 12 degrees C for two hours.

E. coli HB101 competent cells prepared by conventional methods (see, e.g., Mandel and Higa, supra) were transformed with 12.5 µg DNA/ml of cells. Colonies were selected on standard L-broth plates containing 30 ug/ml ampicillin. Plasmid DNA from individual transformant colonies was prepared by conventional methods (see, e.g., Holmes and Quigley, supra). Analytical restriction endonuclease digestions were performed with SalI and HindIII restriction endonucleases. Clones containing plasmids of a size equal to fragments A and B combined were selected for further experimentation. The plasmid was called YpGX3.

## Example IV
## Construction of the Plasmid YpGX4

Plasmid YpGX11 (pJD102, Citron, B.D., Feiss, M. and Donelson, J.E., Gene, 6: 251-269 [1979]) containing a portion of the gal gene cluster with the GAL 1 promoter and regulatory region and GAL 1 gene was digested with restriction endonuclease EcoRI at a concentration of 250 µg/ml in 100 mM NaCl, 50 mM Tris (pH 7.4), 10 mM $MgSO_4$, 0.1 mM DTT, 100 ug/ml BSA, 160 units/ml EcoRI (Bethesda Research Laboratories) for two hours at 37 degrees C. The reaction generates three linear fragments. The middle fragment by size (approximately 1970 base pairs) was isolated by agarose gel electrophoresis and electroelution. The DNA was precipitated in ethanol and resuspended in TE buffer (10 mM Tris Cl (pH 8), 1 mM EDTA (pH 8.0)) and digested with restriction endonuclease RsaI at a concentration of 60 $\mu$g/ml in 50 mM NaCl, 10 mM Tris (pH 7.4), 10 mM $MgSO_4$, 0.1 mM DTT, 100 $\mu$g/ml BSA, and 100 units/ml RsaI (New England Biolabs) for two hours at 37 degrees C. The desired fragment (approximately 483 base pairs) was isolated by agarose gel electrophoresis and electroelution. The fragment, termed "fragment C' was precipitated in ethanol, and contained the GAL 1 promoter and regulatory region plus a small portion of the GAL 1 gene.

YpGX3 DNA was linearized with restriction endonuclease SalI at a concentration of 500 $\mu$g/ml NaCl, 50 mM Tris (pH 7.4), 10 mM $MgSO_4$, 0.1 mM DTT, 100 $\mu$g/ml BSA, and 60 units/ml SalI (Boehringer Mannheim). An aliquot (0.5 $\mu$g DNA was examined by agarose gel electrophoresis to determine completion of the reaction. The DNA was purified by phenol chloroform extraction and precipitation with ethanol.

The SalI linearized YpGX3 DNA was blunted with mung bean nuclease (35 units) at a concentration of 500 $\mu$g/ml in 30 mM sodium acetate (pH 4.6), 50 mM NaCl, 1 mM ZnCl, and 5 percent glycerol for 30 minutes at 30 degrees C. The reaction was terminated by adjusting buffer concentration to 6 mM Tris (pH 8.0), 0.6 mM EDTA, 60 mM NaCl and immediately adding Tris (pH 10) to a final concentration of 13 mM. DNA was extracted and precipitated as previously described. This fragment is now termed "fragment D".

Ligation of fragments C and D was performed at concentrations of 10 $\mu$g/ml and 30 $\mu$g/ml, respectively, in 10 mM DTT, 1 mM ATP, 10 mM $MgCl_2$, 50 mM Tris (pH 7.4), and 150 units/ml T4 DNA ligase (New England Biolabs).

E. coli strain HB101 was transformed by conventional methods (see, e.g., Mandel and Higa, supra).

Transformants which were ampicillin resistant were screened for insertion of fragment C into fragment D by restriction endonuclease analysis and Grunstein-Hogness (Proc. Natl. Acad. Sci., U.S.A., 72: 3961) hybridization using radioactive phosphorous end labeled fragment C as a probe. Plasmids containing the desired configuration were utilized for transformation of Saccharomyces cerevisiae.

## Example V
### Transformation of "Saccharomyces cerevisiae"

Yeast strain DC5 (MAT a, leu 2-3, leu 2-12, his 3, can 1-11) and other strains were transformed by conventional methods (see, e.g., Hinnen et al., Proc. Natl. Acad. Sci. U.S.A., 75, 1929-1933). Two percent glucose was the carbon source in the regeneration medium.

The transformants were then placed on various media and expression of the fusion prochymosin was observed. Two examples of media which were used are:

1. YNB gal his (0.67% Yeast Nitrogen Base, 2% galactose, 20 mg/ml histidine).
2. YP gal (1% Yeast extract, 1% Bacto Peptone, 2% galactose). This medium does not select for plasmid maintenance.

Saccharamyces cerevisiae strain YpGX4 was deposited as NRRL No. Y-15274

## Example VI
### Construction of the Plasmid YpGX31
### Containing the GAL 4 gene

Methods for cloning and identification of the yeast GAL 4 gene have been described (Laughon, A. and R.F. Gesteland (1982) Proc. Natl. Acad. Sci. USA 79, 6287; Johnston, S.A. and J.E. Hopper (1982) Proc. Natl. Acad.

Sci. USA 79, 6971). The procedure of Laughon and Gesteland is being followed with the exception that the spheroplast regeneration medium contained 2% galactose and 0.2% glucose as the carbon sources. The yeast nuclear library is generated by BamHI digest of strain YGX308 and is cloned at the unique BamHI site of YpGX1. This permits excision of the cloned genetic material with BamHI restriction endonuclease. Yeast strain YGX432 (MAT2, leu2-3, leu2-112, gal 4) is transformed with the cloned genomic library and spheroplasts regeneration is conducted at 30°C. Only those cells which are able to metabolize galactose will form large colonies in this medium. Identity of the GAL 4 clone is determined by retransformation of GAL 4 yeast strains and by restriction endonuclease analysis to permit comparison with the published restriction maps for the GAL 4 gene. This vector will be called YpGX40. The cloned GAL 4 is removed from YpGX40 by BamHI digest, blunted with mung bean nuclease, and cloned into the unique PvuII site of YpGX4.

The vector generated in this methodology is called YpGX31. This plasmid contains one copy of the GAL 4 gene with its promoter and the GAL 1 prochymosin fusion gene.

### Example VII
#### Construction of the Plasmid YpGX32 Containing the GAL 81 allele of the GAL 4 gene.

The protocol described in Example VI is being followed exactly for GAL 81 allele cloning with the exception that the yeast genomic library is prepared from strain YGX331 which contains the GAL 81 form of the GAL 4 gene.

22

## Example VIII
### Construction of a Host Yeast Strain Which Provides a Galactose Constitutive, Glucose Repression Resistant Nuclear Background

Yeast strains G183-4A (MAT a, gal 80-2) and GX308 (MATα leu2-3,112 his4) was mixed in YPD medium (1% yeast extract, 1% bactopeptone, 2% glucose) and incubated at 30°C with shaking for four hours. Zygotes were removed from the mating mixture by micromanipulation. Zygotes were isolated since strain G183-4A does not have a selectable nuclear marker. The diploid clones were then sporulated by conventional methods (see, e.g., Sherman, Fink and Lawrence, eds., Methods in Yeast Genetics, Laboratory Manual, New York: Cold Spring Harbor Laboratory (1979).

The spores were released from asci by glusulase treatment and plated on solid YPD medium. The colonies were then screened for the leu 2-3, leu 2-112 mutations. Several haploid spore progeny were transformed with YpGX4 and analyzed for the presence of the fusion prorennin when cells are grown with glucose as the carbon source, albeit at reduced levels. This is the expected result for a strain containing the gal 80 allele.

One of these haploids was then placed on solid medium containing 1% yeast extract, 1% bactopeptone, 2% galactose, 0.1% 2-deoxyglucose, and 2% agar. Several spontaneous mutants resistant to 2-deoxyglucose were obtained and these were transformed with YpGX4 and fusion prochymosin levels were measured by "Western" blot analysis when the cells were grown with 2% glucose or 2% galactose as the carbon sources. Several of these mutants accumulated the same level of fusion prochymosin on these carbon sources. One such mutant strain has been designated GX2A(4) and deposited as NRRL No. Y-15435.

## Example IX
### Integration of linearized Plasmids into Yeast Host Chromosome

Each of the vectors YpGX4, YpGX30, and YpGX31 contain unique restriction endonuclease sites which permit linearization of the molecule. Linear DNA molecules can be utilized for yeast transformation. We have found that for YpGX4 as an example, this linear DNA can survive by two mechanisms in the cell. Recircularization is the most common mechanism to assure replicative capabilities. A second mechanism involves integration of the plasmid genetic information into a pre-existing yeast chromosome. The preferred site used for linearization of plasmid YpGX4 is an XhoI site, adjacent to the cloned LEU 2 gene. By genetic analysis we have shown that integration of the XhoI cut YpGX4 DNA can occur at the LEU 2 locus on chromosome 3 or elsewhere in the genome. The variability in integration site may be the result of the presence of a yeast delta sequence at the XhoI site. Since delta sequences are repeated many times in the yeast genome, chromosome homology at several sites is provided. This result has provided the capability of generating yeast strains with multiple integrated copies of the recombinant DNA derived genetic information. An S. cerevisiae strain in which the XhoI cut YpGX4 DNA has been integrated into chromosome 3 has been designated GX6787 and deposited as NRRL No. Y-15434.

CLAIMS

1. A method of expressing foreign genes in yeast comprising transformation of yeast cells by introduction therein of a cloning vector containing a foreign gene, and yeast galactokinase gene regulatory region and promoter in position to regulate the expression of the foreign gene, and cultivating said transformed yeast cells in a nutrient medium under protein-producing conditions.

2. The method of Claim 1 wherein said yeast is Saccharomyces cerevisiae.

3. The method of Claim 2 wherein said vector is a plasmid capable of replication in Saccharomyces cerevisiae and Escherichia coli.

4. The method of Claim 3 wherein additionally said plasmid is linearly integrated into a host chromosome of said transformed yeast under recombination conditions.

5. The method of Claim 3 wherein said foreign gene is the nucleotide sequence coding for bovine calf prochymosin.

6. The method of Claim 4 wherein said foreign gene is the nucleotide sequence coding for bovine calf prochymosin.

7. The method of Claim 5 wherein the nucleotide sequence coding for bovine calf prochymosin is modified by the insertion of a Sal I linker at the second codon position of said nucleotide sequence.

8. The method of Claim 6 wherein the nucleotide sequence coding for bovine calf prochymosin is modified by the insertion of Sal I linker at the second codon position of said nucleotide sequence.

9. A cloning vector containing a foreign gene and yeast galactokinase gene regulatory region and promoter in position to regulate the expression of the foreign gene.

10. The plasmid, YpGX4, comprising the RsaI fragment of the yeast gal gene cluster containing the GAL 1 promoter and regulatory region and small portion of the GAL 1 gene; the nucleotide sequence coding for bovine calf prochymosin on the 3' proximal side of said EcoRI fragment, wherein said prochymosin sequence contains a Sal I linker at the second codon position; a yeast transcription termination sequence on the 3' proximal side of said prochymosin nucleotide sequence; the gene coding for LEU 2 on the 3' proximal side of said yeast transcription terminator; the gene coding for amp$^R$ on the 3' proximal side of said LEU 2 gene; a yeast origin of replication derived from the yeast plasmid 2 micron circle; and an E.coli origin of replication derived from the plasmid pBR322.

11. A microorganism transformed with the plasmid of Claim 10.

12. S. cerevisiae strain YpGX4, deposited as NRRL No.Y-15274.

13. S.cerevisiae strain GX6787, deposited as NRRL No. Y-15434, and containing the plasmid YpGX4 linearly integrted into host chromosome 3.

14. S. cerevisiae strain GX2A(4), deposited as NRRL No. Y-15435, containing the plasmid YpGX4, and capable of expressing a bovine calf prochymosin fusion product on a glucose containing nutrient medium.

15. The protein of a foreign gene produced by the method of any one of claims 1 to 4.

16. The method of claim 3 or claim 4, wherein the said plasmid is the plasmid of claim 10.

17. GAL 1 - bovine calf prochymosin fusion protein comprising bovine calf prochymosin having attached thereto terminal sequence of galactokinase produced by the method of claim 16.

0128743

1/2

FIG. 1.

YEpl3
10,700 bp

Leu 2 — SalI
Xho I→
YEAST TRANSCRIPTION
TERMINATOR (YTT)
Hind III
Hind III
amp R
SalI tet R

FIG. 2.

YpGX1

Leu 2
Xho I→
YEAST TRANSCRIPTION
TERMINATOR
Hind III
Hind III
amp R
SalI tet R

FIG. 4.

YpGX3

Xho I
YTT
Hind III
MODIFIED BOVINE CALF
PROCHYMOSIN GENE
SalI

FIG. 5.

YpGX4

Xho I
YTT
Hind III
MODIFIED BOVINE CALF PROCHYMOSIN
GENE
GAL I PROMOTER AND REGULATORY
REGION PLUS SMALL SEGMENT OF GAL I GENE
DNA
RLS
SLS

FIG. 3.